Europäisches Patentamt

European Patent Office

Office européen des brevets

(11) Veröffentlichungsnummer: **0 167 058**
A2

# (12) EUROPÄISCHE PATENTANMELDUNG

(21) Anmeldenummer: 85107546.5

(22) Anmeldetag: 19.06.85

(51) Int. Cl.⁴: **C 12 P 13/22**
//(C12P13/22, C12R1:645, 1:01, 1:19, 1:465)

(30) Priorität: 29.06.84 DE 3423936

(43) Veröffentlichungstag der Anmeldung:
08.01.86 Patentblatt 86/2

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Voelskow, Hartmut, Dr.
Akazienstrasse 22
D-6234 Hattersheim am Main(DE)

(72) Erfinder: Keller, Reinhold, Dr.
Wiesenweg 5
D-6232 Bad Soden am Taunus(DE)

(72) Erfinder: Schlingmann, Merten, Dr.
Schneidhainer Strasse 32a
D-6240 Königstein/Taunus(DE)

(72) Erfinder: Platen, Martin, Dr.
Krautgartenweg 6
D-6000 Frankfurt am Main 50(DE)

(72) Erfinder: Then, Johann, Dr.
Sulzbacher Weg 2
D-6238 Hofheim am Taunus(DE)

(72) Erfinder: Wöhner, Gerhard, Dr.
Flörsheimer Strasse 27
D-6093 Flörsheim am Main(DE)

(54) Verfahren zur Herstellung von L-Phenylalanin.

(57) L-Phenylalanin läßt sich mit Mikroorganismen der Reihe E. coli, Paracoccus denitrificans, Torula, Rhodotorula oder Streptomyces nach Adaptation an Phenylbrenztraubensäure durch Aminierung mit geeigneten Stickstoffquellen herstellen. Vorteilhaft wird der Mikroorganismus in Form fixierter Zellen eingesetzt.

EP 0 167 058 A2

Verfahren zur Herstellung von L-Phenylalanin

Phenylalanin ist eine Aminosäure, die in jedem lebenden Organismus vorkommt. Die meisten Bakterien und Pilze, einschließlich der Hefen, synthetisieren das zum Wachstum notwendige Phenylalanin selbst. Die dazu notwendigen Enzymsysteme sind aber nur mit so geringer Aktivität vorhanden, daß nicht mehr Phenylalanin gebildet wird als die Zelle gerade benötigt.

Die langsamsten Schritte in der Biosynthese von Phenylalanin liegen im Aufbau des aromatischen Ringes. Es wurde deshalb schon versucht, aromatische Vorstufen durch Biotransformation mit Rhodotorula-Hefen oder E. coli in Phenylalanin zu überführen. Die Ausbeuten waren allerdings so gering, daß sie für eine technische Produktion nicht in Frage kamen.

Es wurde schon vorgeschlagen (nicht vorveröffentlichte deutsche Offenlegungsschrift 3 427 495), L-Aminosäuren aus α-Ketosäuren dadurch herzustellen, daß die gewünschte α-Ketosäure Mikroorganismen während der logarithmischen Wachstumsphase zugeführt wird. Phenylbrenztraubensäure wird in diesem Sinne von Brevibacterium in l-Phenylalanin umgesetzt.

Auf viele Mikroorganismen, unter anderem auch auf die erfindungsgemäß eingesetzten, wirkt Phenylbrenztraubensäure jedoch toxisch. Daher war überraschend, daß durch Adaptation an steigende Mengen Phenylbrenztraubensäure Mikroorganismen selektiert werden konnten, die mit dieser Vorstufe bis zu etwa 15 g L-Phenylalanin pro Liter Kulturmedium produzieren.

Die Erfindung betrifft somit ein Verfahren zur Herstellung von L-Phenylalanin aus Phenylbrenztraubensäure und

- 2 -

einer Stickstoffquelle für die Aminierung mit Hilfe von Mikroorganismen, das dadurch gekennzeichnet ist, daß man einen Mikroorganismus aus der Reihe E. coli, Paracoccus denitrificans, Torula, Rhodotorula oder Streptomyces an Phenylbrenztraubensäure bzw. Phenylpyruvat adaptiert.

Bevorzugte Mikroorganismenstämme sind E. coli ATCC e 11303, Paracoccus denitrificans DSM 65, Rhodotorula glutinis DSM 70398 sowie verschiedene, aus Erdproben isolierte Streptomyceten.

Die Adaptation geeigneter Stämme erfolgt durch wiederholte Zugabe einer gleichbleibenden Menge oder durch Zugabe steigender Mengen Phenylbrenztraubensäure zu den entsprechenden Anzuchtmedien. Vor oder während der Selektion gegen diesen aromatischen Ausgangsstoff kann vorteilhaft zur Beschleunigung der Adaptation auch eine Mutation der Mikroorganismen nach an sich bekannten Methoden erfolgen. Hierdurch und durch Optimierung der verwendeten Medien und Wachstumsbedingungen können die ursprünglich erzielten Phenylalanin-Ausbeuten von üblicherweise etwa 0,01 bis 0,5 g/l, in Sonderfällen bis etwa 2,4 g, auf bis zu 15 g/l gesteigert werden.

Vorteilhaft werden die Mikroorganismen in fixierter Form eingesetzt. Für die Fixierung kommen die bekannten Verfahren in Betracht, vorteilhaft die Methoden nach den deutschen Offenlegungsschriften 3 237 341 und 3 243 591 sowie die darin genannten Verfahren. Die so immobilisierten Biokatalysatoren können in einem Reaktor nach dem Schlaufenprinzip, insbesondere in einem Airliftreaktor, eingesetzt werden, wie es in der deutschen Offenlegungsschrift 3 247 214 vorgeschlagen wurde.

Als Stickstoffquelle werden einerseits Harnstoff sowie Ammoniak in Form seiner Salze, insbesondere Salze von Mono- und Polycarbonsäuren, und andererseits Aminosäuren, insbesondere Glycin, Asparagin, Asparaginsäure, Glutamin oder Glutaminsäure, bevorzugt.

Phenylbrenztraubensäure kann in Form der freien Säure oder geeigneter Salze (entsprechend dem verwendeten Medium) eingesetzt werden. Ihre Zugabe kann gleich zu Beginn der beimpften Mikroorganismenkultur oder nach einer Wachstumsphase von etwa 10 bis 90 Stunden, vorzugsweise 24 bis 48 Stunden, in einer Menge von etwa 0,1 bis 2 %, bezogen auf das Gewicht des Mediums, erfolgen. Dieser Befund ist ebenfalls überraschend, da nach der deutschen Offenlegungsschrift 3 427 495 die beste Ausbeute an eine Zugabe von α-Ketosäure während der logarithmischen Wachstumsphase gebunden ist. Bezogen auf die Stickstoffquelle wird die Phenylbrenztraubensäure in einem geringen stöchiometrischen Unterschuß eingesetzt. Mit diesen Ausgangsmaterialien werden die Kulturen etwa 10 bis 90 Stunden weiter bebrütet, vorzugsweise 12 bis 48 Stunden, jeweils angepaßt an die eingesetzten Stämme. Man kann die Ausgangsmaterialien während der Wachstumsphase auch in geringerer Konzentration, etwa 0,02 bis 0,5 %, einsetzen, worauf dann gegen Ende oder nach der Wachstumsphase, etwa im Lauf von 24 bis 48 Stunden, die weitere Zugabe in der vorstehend angegebenen Menge erfolgt. Die günstigste Vorgehensweise für den jeweiligen Stamm kann in einfachen Vorversuchen ermittelt werden.

Die Zugabe von Fumarsäure bzw. deren Salzen zum Fermentationsmedium hat einen positiven Einfluß auf die Umsetzung von Phenylbrenztraubensäure zu L-Phenylalanin. Besonders vorteilhaft ist die Zugabe des Ammoniumsalzes, da dann die Ammoniumionen als Stickstoffdonor für die Umsetzung dienen können.

Die Optimierung der verwendeten Medien und Wachstumsbedingungen, beispielsweise pH-Wert, Sauerstoffversorgung, Temperatur, Konzentrationen der Ausgangsmaterialien und gegebenenfalls Fixierungsbedingungen ist ebenfalls anhand einfacher Vorversuche leicht möglich.

Die nach dem erfindungsgemäßen Verfahren gezüchteten freien
Zellen können immobilisiert werden und produzieren dann
bei kontinuierlicher Zugabe von Nährlösung mit guter Raum-
Zeit-Ausbeute L-Phenylalanin. Eine bevorzugte Ausführungsform ist die Immobilisierung nach vollendeter L-Phenylalaninsynhtese im "batch"-System. Es können dann noch bis
zu etwa 10 g Phenylalanin pro 1 Nährlösung über längere
Zeit kontinuierlich synthetisiert werden.

In den folgenden Beispielen beziehen sich Prozentangaben
auf das Gewicht.

Beispiel 1

Escherichia coli ATCC e 11303 wurde mit N-Methyl-N'-nitro-
N-nitrosoguanidin in an sich bekannter Weise mutiert.
Die behandelten Zellen wurden als 3 %iges Inokulum in
100 ml des Adaptationsmediums der Zusammensetzung

1,5  % Fumarsäure
2,0  % Fleischextrakt
0,05 % $MgSO_4 \cdot 7 H_2O$
1,75 % Ammoniak
0,2  % $KH_2PO_4$ und

steigenden Mengen von Phenylpyruvat und Asparaginsäure
gegeben und bei einem pH-Wert von 7,5 und einer Temperatur
von 37°C auf einer Schüttelmaschine 48 Stunden inkubiert. Die Kulturen wurden mehrfach überimpft, wobei
die Mengen von Phenylpyruvat und Asparaginsäure nach
den in Tabelle 1 angegebenen Werten gesteigert wurden.

Tabelle 1

| Zahl der | Zugabe [g] | |
| --- | --- | --- |
| Überimpfungen | Phenylpyruvat | Asparaginsäure |
| erste Überimpfung | 5 | 4 |
| zweite Überimpfung | 10 | 7 |
| dritte Überimpfung | 16 | 10 |
| vierte Überimpfung | 24 | 20 |

Anschließend wurden die Kulturen um den Faktor $10^5$ bis $10^6$ mit physiologischer Kochsalzlösung verdünnt und auf Platten ausplattiert, die ein Medium mit obiger Zusammensetzung enthielten, zuzüglich 16 g/l Asparaginsäure, 24 g/l Phenylpyruvat und 15 g/l Agar. Nach Inkubation bei 37°C über einen Zeitraum von 1 bis 2 Tagen wurden gut gewachsene, große Kolonien ausgewählt. Auf diese Weise wurden unter anderem die Mutanten Escherichia coli E-196 und Escherichia coli E-197 isoliert.

Beispiel 2

Gemäß Beispiel 1 mutierte und selektionierte Kulturen von E. coli ATCC e 11303, sowie der Wildstamm als Kontrolle, wurden in 100 ml des folgenden Mediums angeimpft:

1,5  % Fumarsäure,
2,0  % Fleischextrakt,
0,05 % $MgSO_4 \cdot 7 H_2O$,
1,75 % Ammoniak,
0,2  % $KH_2PO_4$,
0,7  % Asparaginsäure und
1    % Phenylbrenztraubensäure als Na-Salz und
0,01 % $CaCl_2 \cdot 2 H_2O$

Nach 2 Tagen wurde daraus mit 3 % Inokulum wieder eine gleiche Kultur angesetzt und ebenso zwei weitere Male nach je 2 Tagen. Die Kulturen wurden auf einer Schüttelmaschine bei 37°C bebrütet. Aus der letzten Kultur wurde ein neuer Ansatz von 4 l, verteilt in 8 Erlenmeyerkolben (2 l Bruttovolumen) mit je 500 ml Füllung, mit dem gleichen Medium beimpft und bei 37°C bebrütet. Nach 2 Tagen wurden die in Tabelle 2 aufgeführten Phenylalaningehalte erreicht.

Tabelle 2

| Mikroorganismus | Phenylalanin im Medium (g/l) |
|---|---|
| E. coli E-196 | 9,3 |
| E. coli E-197 | 7,5 |
| E. coli ATCC e 11303 (Wildstamm) | 2,4 |

Bei Erhöhung der Konzentration von Phenylpyruvat auf 1,7 %
und der Konzentration von Asparaginsäure auf 1 % wurde ein
Phenylalaningehalt von 15 g/l erzielt. Die Zellen wurden
nach 2 Tagen durch Zentrifugation abgeerntet und nach dem
Verfahren der deutschen Offenlegungsschrift 3 237 341,
Beispiel 1, immobilisiert.

Beispiel 3

Eine erfindungsgemäß selektionierte Kultur von Paracoccus
denitrificans DSM 65 wurde mit einer Impföse von einer
Schrägagarkultur auf 100 ml des folgenden Mediums beimpft:

1    % Glucose,
0,4  % Caseinpepton,
0,4  % Fleischextrakt,
0,05 % Hefeextrakt,
0,05 % Leberextrakt,
0,25 % Kochsalz und
0,15 % $MgSO_4 \cdot 7 H_2O$.

Die Kultur wurde in einem Erlenmeyerkolben (Bruttovolumen
300 ml) auf einer Schüttelmaschine bei 30°C bebrütet. Nach
2 Tagen wurde daraus ein Laborfermenter mit 10 l Füllvolumen mit dem gleichen Medium beimpft. Nach 24 Stunden Anwachszeit bei 500 upm und 10 l Luft/min und einer
Temperatur von 30°C wurden je 50 g Glycin und Phenylpyruvat zugegeben. Nach zwei weiteren Tagen erreichte die
Kultur eine optische Dichte (Extinktion bei 580 nm) von
6,2. Das Phenylpyruvat wurde dabei zu 56 % in Phenylalanin umgewandelt.

Die Kultur wurde durch Zentrifugation abgeerntet und die
Zellen nach dem Verfahren der deutschen Offenlegungsschrift 3 237 341, Beispiel 2, immobilisiert.

Beispiel 4

50 Streptomyces sp.-Isolate aus Erdbodenproben wurden jeweils in 100 ml des folgenden Mediums überimpft:

2   % Sojamehl,

2   % Mannit,

0,5  % Phenylbrenztraubensäure als Na-Salz und

0,5  % Asparaginsäure.

Zusätzlich wurden die Stämme in 100 ml eines entsprechenden Mediums überimpft, das an Stelle der Asparaginsäure Glycin enthielt. Die Kulturen wurden 5 Tage auf einer Schüttelmaschine bei 30°C bebrütet. Proben wurden nach 3 und 5 Tagen entnommen. 7 Stämme aus den getesteten Kulturen setzten Phenylpyruvat zu 30 % bis 60 % in Phenylalanin um, so daß sich diese für die weitere Adaptation eigneten.

Beispiel 5

100 g der nach Beispiel 2 erhaltenen immobilisierten Zellen wurden in eine auf 30°C thermostatisierte Glassäule gegeben und mit einer Substratlösung der Zusammensetzung

16 g/l Phenylbrenztraubensäure als Na-Salz,

10 g/l L-Asparaginsäure,

10 mg/l $MgSO_4 \cdot 7 H_2O$ und

2 mg/l Pyridoxalphosphat

versetzt. Bei einer Durchflußgeschwindigkeit von 50 ml/Std., einem pH von 7,5 und 30°C erreichte man eine Konzentration an L-Phenylalanin von 9,8 g/l. Frei werdende Oxalessigsäure decarboxylierte hierbei zu etwa 50 % in Brenztraubensäure. Die Produktlösung wurde durch Abdestillieren von Wasser auf die Hälfte aufkonzentriert, der pH-Wert auf 5,5 eingestellt und durch Abkühlen auf 5°C das L-Phenylalanin zur Kristallisation gebracht.

Beispiel 6

100 g der nach Beispiel 2 erhaltenen immobilisierten
Zellen wurden in eine auf 30°C thermostatisierte Glassäule gegeben. Eine Substratlösung der Zusammensetzung

10 g/l Phenylbrenztraubensäure als Na-Salz,
10 g/l Ammoniumfumarat,
10 mg/l $MgSO_4 \cdot 7 H_2O$ und
2 mg/l Pyridoxalphosphat

wurde bei einem pH-Wert von 8,0 um 30°C mit einer Durchflußgeschwindigkeit von 20 ml/Std. über die Säule gepumpt.
Es bildeten sich dabei 9,7 g/l L-Phenylalanin. Durch Aufkonzentrieren und Auskristallisieren gemäß Beispiel 5
wurde das L-Phenylalanin in hoher Reinheit gewonnen.

Beispiel 7

100 g der nach Beispiel 2 durch Zentrifugation geernteten
Zellen wurden mit 80 ml einer 8 %igen Natriumalginatlösung
verrührt und durch eine Düse in ein Vernetzerbad, bestehend
aus 0,5 m Kalziumchloridlösung, getropft. Die erhaltenen
Kügelchen wurden abfiltriert, in eine Glassäule gegeben
und mit einer Substratlösung gemäß Beispiel 5 versetzt.
Bei einer Durchflußgeschwindigkeit von 70 ml/Std. wurden
9,8 g/l L-Phenylalanin erhalten.

Beispiel 8

100 g der nach Beispiel 3 erhaltenen immobilisierten
Zellen wurden in eine auf 30°C thermostatisierte Glassäule gegeben und mit einer Substratlösung der Zusammensetzung

10 g/l Phenylbrenztraubensäure,
5 g/l Glycin,

10 mg/l $MgSO_4$ · 7 $H_2O$ und
 3 mg/l Pyridoxalphosphat

versetzt. Bei einem pH-Wert von 7,0 und einer Durchflußgeschwindigkeit von 20 ml/Std. erhielt man 9,6 g/l L-
Phenylalanin.

Patentansprüche:

1. Verfahren zur Herstellung von L-Phenylalanin aus Phenylbrenztraubensäure und einer Stickstoffquelle für die Aminierung mit Hilfe von Mikroorganismen, dadurch gekennzeichnet, daß man einen Mikroorganismus aus der Reihe E. coli, Paracoccus denitrificans, Torula, Rhodotorula oder Streptomyces an Phenylbrenztraubensäure adaptiert.

2. Verfahren nach Anspruch 1, dadurch gekennzeichnet, daß vor oder während der Selektion der Mikroorganismen gegen Phenylbrenztraubensäure eine Mutation durchgeführt wird.

3. Verfahren nach Anspruch 1 oder 2, dadurch gekennzeichnet, daß als Mikroorganismus E. coli ATCC e 11303, Paracoccus denitrificans DSM 65, Rhodotorula glutinis DSM 70398 oder ein aus Erdproben isolierter Streptomycet eingesetzt wird.

4. Verfahren nach einem oder mehreren der vorhergehenden Ansprüche, dadurch gekennzeichnet, daß als Stickstoffquelle ein Mono- oder Polycarbonsäuresalz des Ammoniaks, Glycin, Asparagin, Asparaginsäure, Glutamin oder Glutaminsäure eingesetzt werden.

5. Verfahren nach einem oder mehreren der vorhergehenden Ansprüche, dadurch gekennzeichnet, daß der Mikroorganismus gegen steigende Mengen Phenylbrenztraubensäure selektiert wird.

6. Verfahren nach einem oder mehreren der vorhergehenden Ansprüche, dadurch gekennzeichnet, daß der Mikroorganismus in Form immobilisierter Zellen eingesetzt wird.

7. Verfahren nach einem oder mehreren der vorhergehenden Ansprüche, dadurch gekennzeichnet, daß Fumarsäure oder deren Salze der Nährlösung zugesetzt werden, in der die Umsetzung zu Phenylalanin stattfindet.